Europäisches Patentamt

European Patent Office

Office européen des brevets

(19)

(11) Numéro de publication : **0 401 109 B1**

(12)

# FASCICULE DE BREVET EUROPEEN

(45) Date de publication du fascicule du brevet :
**29.09.93 Bulletin 93/39**

(51) Int. Cl.⁵ : **C07K 15/10,** A61K 37/64, **C07K 7/10**

(21) Numéro de dépôt : **90401432.1**

(22) Date de dépôt : **30.05.90**

(54) Microprotéines, procédé de préparation et application à titre de médicaments de ces microprotéines.

(30) Priorité : **31.05.89 FR 8907155**

(43) Date de publication de la demande :
**05.12.90 Bulletin 90/49**

(45) Mention de la délivrance du brevet :
**29.09.93 Bulletin 93/39**

(84) Etats contractants désignés :
**AT BE CH DE DK ES FR GB GR IT LI LU NL SE**

(56) Documents cités :
**MEDLINE ABSTRACT, no. 89274165; A. HEITZ et al.**
**MEDLINE ABSTRACT, no. 89233599; A. FAVEL et al.**
**BIOLOGICAL ABSTRACT, no. 37096274; D. LE NGUYEN et al.**
**MEDLINE ABSTRACT, no. 89350964; D. LE NGUYEN et al.**

(73) Titulaire : **ROUSSEL-UCLAF**
**35, Boulevard des Invalides**
**F-75007 Paris (FR)**

(72) Inventeur : **Castro, Bertrand**
**L'Estaple, Avenue des Costières**
**F-34130 Mauguio (FR)**
Inventeur : **Favel, Anne**
**67 rue de la Loubière**
**F-13005 Marseille (FR)**
Inventeur : **Le-Nguyen. Dung**
**391 Le Grand Mail**
**F-34080 Montpellier (FR)**
Inventeur : **Previero, Maria-Antonia**
**Mas de la Droleta, 607, rue de Père Prévost**
**F-34100 Montpellier (FR)**

(74) Mandataire : **Vieillefosse, Jean-Claude et al**
**Roussel-Uclaf 111, route de Noisy B.P. 9**
**F-93230 Romainville (FR)**

Jouve, 18, rue Saint-Denis, 75001 PARIS

## Description

La présente demande concerne de microprotéines, le procédé de préparation et l'application à titre de médicament de ces microprotéines.

On sait que les semences d'Ecballium elaterium plus communément connu sous le nom de "cornichon sauteur" sont une source très importante de protéines inhibant les proteinases. On a ainsi notamment isolé de ces semences une microprotéine connue sous le nom d'EETI II (Ecballium elaterium Trypsin Inhibitor II) de structure :

G C P R I L M R C K Q D S D C L A G C V C G P N G F C G

4 5 7 10 15 20 25

(- Protease inhibitors from Ecballium Elaterium seeds. A. Favel, H. Mattras, M.A. Coletti-Previero, R. Zwilling, E.A. Robinson Castro. International Journal of Peptide & Protein Research.
- 1H 2D NMR and Distance Geometry of the folding of Ecballium elaterium, a member of the squash inhibitor family. A. Heitz, L. Chiche, D. Le-Nguyen B. Castro, Biochemistry, (1989) 28, p. 2392, 2398.)

La nomenclature utilisée est celle de la commission IUPAC-IUB (1984) European J. Biochem 183, 9-37.

Cette microprotéine qui renferme 28 résidus d'amino-acides, 3 ponts disulfures et 5 résidus de glycine présente notamment la propriété d'inhiber la trypsine.

En essayant de remplacer certains restes d'amino-acides de cette microprotéine par d'autres amino-acides, la demanderesse a préparé par synthèse de nouvelles microprotéines dans lesquelles l'activité anti-trypsine de l'EETI II a disparu au profit d'une activité anti-élastasique tant contre l'élastase pancréatique bovine que contre l'élastase leucocytaire humaine. Ces produits présentent en outre l'intérêt de pouvoir être préparés par synthèse totale en grandes quantités.

La présente demande a ainsi pour objet de nouvelles micro-protéines renfermant 28 amino-acides répondant à la formule générale (I) :

G C P-X-Y-L-Z R C K Q D S D C L A G C V C G P N G F C G

4 5 7 10 15 20 25

dans laquelle :
- X en position 4 représente un résidu de valine, de leucine, d'isoleucine, de norleucine, d'alanine, de cyclohexylalanine, d'o-méthylthéonine, de phénylglycine, d'acide alpha-amino butyrique ou d'arginine,
- Y en position 5 représente un résidu d'isoleucine ou de serine,
- Z en position 7 représente un résidu de méthionine ou de norleucine, étant entendu que Z représente un résidu de norleucine lorsque X représente un résidu d'arginine.

Parmi les microprotéines telles que définies ci-dessus, on retient notamment celles caractérisées en ce que, Y en position 5 représente un résidu d'isoleucine, X en position 4 et Z en position 7 ont la signification déjà indiquée.

Parmi ces dernières, on retient de préférence les microprotéines dans lesquelles Z en position 7 représente un résidu de norleucine, ainsi que celles dans lesquelles X en position 4 représente un résidu de valine, d'alanine ou de norleucine.

Parmi les microproteines préférées de l'invention, on retient tout particulièrement celles dans lesquelles :
- X en position 4 représente un résidu de valine, Y en position 5 représente un résidu d'isoleucine et Z en position 7 représente un résidu de norleucine,
- X en position 4 représente un résidu d'alanine, Y en position 5 représente un résidu d'isoleucine et Z en

2

position 7 représente un résidu de méthionine,

- X en position 4 représente un résidu de norleucine, Y en position 5 représente un résidu de sérine et Z en position 7 représente un résidu de méthionine.

L''invention a également pour objet un procédé de préparation des microprotéines telles que définies ci-dessus, caractérisé en ce que l'on effectue une synthèse en phase solide en introduisant séquentiellement sur une résine oxyméthyl polystyrène réticulée les amino-acides dûment protégés en utilisant un agent de couplage, libère de la résine la chaîne peptidique ainsi formée et déprotège les amino-acides, puis, cyclise les 3 ponts disulfurés pour obtenir la microprotéine recherchée.

Dans des conditions préférentielles de mise en oeuvre, le procédé ci-dessus décrit est caractérisé en ce que :

- la résine oxyméthyl polystyrène réticulée est une résine de type "Boc-gly-CM" dans laquelle Boc est un groupement tertiobutyloxycarbonyl, gly est un groupement glycine et CM désigne la résine oxyméthyl polystyrène réticulée,
- l'agent de couplage est le BOP ou (benzotriazol-1-yloxy) tris(diméthylamino) phosphonium hexafluorophosphate,
- la libération de la résine de la chaîne peptidique ainsi que la déprotection des amino-acides est effectuée au moyen de l'acide fluorhydrique en opérant à basse température.

Les microprotéines, objet de la présente invention présentent de très intéressantes propriétés pharmacologiques ; on note en particulier une remarquable activité anti-élastase, tant contre l'élastase pancréatique bovine, que contre l'élastase leucocytaire humaine. L'activité anti-trypsine de ce produit a par contre disparu.

Ces propriétés sont illustrées plus loin dans la partie expérimentale.

Ces propriétés justifient l'utilisation des nouvelles microprotéines répondant à la formule (I) ci-dessus à titre de médicaments.

La présente invention a ainsi également pour objet l'application à titre de médicaments des nouvelles microprotéines telles que définies par la formule générale (I) ci-dessus.

Parmi les médicaments, objet de l'invention, on retient notamment les médicaments caractérisés en ce qu'ils sont constitués par les nouvelles microprotéines répondant à la formule générale (I) ci-dessus dans laquelle Y en position 5 représente un résidu d'isoleucine, X en position 4 et Z en position 7 ont la signification déjà indiquée.

Parmi ces derniers on retient de préférence les médicaments renfermant les microprotéines telles que définies ci-dessus dans lesquelles Z en position 7 représente un résidu de norleucine, ainsi que dans lesquelles X en position 4 représente un résidu de valine, d'alanine ou de norleucine.

Parmi les médicaments préférés de l'invention, on retient tout particulièrement ceux constitués par des microprotéines dans lesquelles :

- X en position 4 représente un résidu de valine, Y en position 5 représente un résidu d'isoleucine et Z en position 7 représente un résidu de norleucine,
- X en position 4 représente un résidu d'alanine, Y en position 5 représente un résidu d'isoleucine et Z en position 7 représente un résidu de méthionine,
- X en position 4 représente un résidu de norleucine, Y en position 5 représente un résidu de sérine et Z en position 7 représente un résidu de méthionine.

Ces médicaments trouvent, par exemple, leur emploi en pneumologie dans le traitement de l'emphysème, des pneumonies, des bronchites, des troubles pulmonaires causés par le tabagisme ou la pollution atmosphérique, en cardiologie dans le traitement de l'athérosclérose, en rhumatologie, dans le traitement des arthrites, ainsi que par exemple en dermatologie dans le traitement du psoriasis, des brûlures, des buloses, et dans le vieillissement de la peau, en gastroentérologie, dans le traitement des pancréatites aigues et d'une manière générale dans le traitement de toutes les affections mettant en cause le disfonctionnement de l'élastase.

La dose usuelle, variable selon le produit utilisé, le sujet traité et l'affection en cause peut être par exemple de 1 à 300 mg par jour par voie intraveineuse chez l'homme.

L'invention a également pour objet les compositions pharmaceutiques qui renferment au moins un dérivé précité à titre de principe actif.

A titre de principe actif, les microprotéines répondant à la formule générale (I), peuvent être incorporées dans des compositions pharmaceutiques destinées à la voie digestive ou parentérale ou topique.

Ces compositions pharmaceutiques peuvent être, par exemple, solides ou liquides et se présenter sous les formes pharmaceutiques couramment utilisées en médecine humaine, comme par exemple, les comprimés simples ou dragéifiés, les gélules, les capsules, les granulés, les suppositoires, les préparations injectables, les pommades, les aérosols ; elles sont préparées selon les méthodes usuelles. Le ou les principes actifs peuvent être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Il va être donné maintenant à titre non limitatif, des exemples de mise en oeuvre de l'invention.

## EXEMPLE 1: Dérivé Val-4, Nle-7

### STADE A : Assemblage de la chaîne peptidique

On utilise la méthode de la synthèse en phase solide avec une résine de oxyméthyl polystyrène réticulée (ci-après CM-résine). On utilise une résine de type "Boc-Gly-CM-résine" dans laquelle Boc est un groupement tertiobutyloxycarbonyl, Gly représente un groupement glycine et CM-résine désigne la résine de oxyméthyl polystyrène réticulée.

La résine renfermant 0,4 mmol Gly/g a été préparée par la méthode du sel de cesium selon la technique décrite par GISIN, BF (1973) Helv. Chim. Acta 56, 1476-1482.

Les groupements protecteurs des acides aminés ont été les suivants :
- p-méthoxy benzyl pour la cysteine,
- ester de cyclohexyl pour l'acide aspartique,
- xanthyl pour l'asparagine et la glycine,
- tosyl pour l'arginine,
- benzyl pour la sérine,
- o-chlorobenzyloxycarbonyl pour la lysine,
- la méthionine n'est pas protégée.

La réaction de couplage a été effectuée en utilisant le BOP (benzotriazol-1-yloxy tris (diméthylamino) phosphonium hexafluorophosphate.

Un cycle de couplage peut être résumé comme suit :

### Déprotection :

1 - Lavage avec de l'acide trifluoroacétique à 30 % dans le dichlorométhane, 1 minute.
2 - Déprotection avec de l'acide trifluoroacétique à 30 % dans le dichlorométhane, 30 minutes.
3 - Lavage avec de l'éthanedithiol à 3 % dans l'isopropanol, une fois.
4 - Lavage au dichlorométhane, 2 fois.

### Couplage :

1 - Addition de complexe Boc-acide aminé et de BOP.
2 - Addition de diisopropyléthylamine (4 équi.....) puis de solvant (dichlorométhane ou diméthylformamide) sous agitation.
3 - Après réaction négative à la ninhydrine lavage 2 fois au dichlorométhane.

Pour l'acétylation la résine est traitée par le mélange (anhydride acétique/dichlorométhane (50/50 V/V) pendant 15 minutes.

Comme la méthionine n'est pas protégée le dernier groupement-Boc est éliminé par l'acide trifluoroacétique avant le traitement par l'acide fluorhydrique pour éviter une tert-butylation.

Au départ de 3 g de résine "Boc-Gly-CM-résine" on a ainsi obtenu en opérant manuellement 9,5 g de complexe chaîne peptidique protégée, résine que l'on utilise directement au stade suivant.

### STADE B : Libération du complexe chaîne peptidique protégée-résine

On fait réagir 3 g de complexe chaîne peptidique protégée-résine pendant 60 minutes avec 30 ml d'acide fluorhydrique à 0°C en présence d'anisole (3 ml) et diméthylsulfide (1 ml). La résine est lavée à l'éther ; le peptide brut est extrait par une solution aqueuse d'acide acétique à 20 %. Après lyophilisation, on obtient 1,1 g de produit brut (non cyclisé) que l'on utilise directement au stade suivant :

### STADE C : Cyclisation et purification

On dissout 400 mg du peptide brut obtenu ci-dessus, dans 350 ml d'eau, agite vigoureusement, ajoute de la diisopropyl éthylamine jusqu'à obtention de pH 8 (une goutte de mélange est recueillie toutes les heures et ajoutée à une goutte d'une solution contenant de l'acide dithiobis (2-nitrobenzoïque) dans un tampon molaire

de $K_2HPO_4$ (pH 8) pour suivre la réaction d'oxidation). Pendant toute la réaction on maintient le pH à 8 par addition de diisopropyléthylamine.

Après 50 heures, on constate l'absence de coloration jaune dans le test à l'acide dithiobis (2-nitrobenzoique).

Le produit obtenu est purifié par chromatographie liquide haute performance (HPLC) sur colonne Whatman M20 OD₃ et élué par une solution $CH_3CN/H_2O$ renfermant 0,1 % d'acide trifluoroacétique.

On lyophilise la fraction obtenue et recueille finalement 40 mg de produit attendu.

La composition en aminoacides figure dans le tableau I ci-après :

## EXEMPLES 2 à 9:

En opérant de la même manière qu'à l'exemple 1 mais avec d'autres amino-acides, on a préparé 8 autres dérivés qui figurent dans le tableau I ci-après.

Les compositions en amino acides sont également indiquées dans le tableau I.

## TABLEAU I

|  | \multicolumn{9}{c}{PRODUITS DES EXEMPLES} |
|---|---|---|---|---|---|---|---|---|---|
|  | 1 | 2 | 3 | 4 | 5 | 6 | 7 | 8 | 9 |
|  | [Val4, Nle7] | [Nle7] | [Ala4, Nle7] | [Ala4] | [Phe4, Nle7] | [Nle4, Nle7] | [Nle4, Ser5, Nle7] | Ser5 | Ile4 |
| Asp | 2,9 | 3 | 3,1 | 3,3 | 2,4 | 2,8 | 3 | 2,8 | 2,9 |
| Glu | 1 | 1 | 1 | 1 | 1 | 1 | 1 | 1,1 | 1,1 |
| Ser | 1 | 0,9 | 1 | 0,9 | 1,1 | 1 | 1,8 | 1,9 | 0,9 |
| Gly | 5,4(6) | 5,7(6) | 6,4(6) | 6,6 | 5,2(6) | 4,7 | 4,25 | 5,9 | 6,1 |
| Arg | 1,1 | 2,1 | 1,2 | 1,2 | 1 | 1,3 | 1,17 | 1,1 | 1,1 |
| Ala | 1 | 1 | 2 | 2 | 1 | 1 | 1 | 1 | 1,1 |
| Pro | 2,25 | 2,1 | 2,75 | 2,6 | 2,1 | 2,2 | 2,1 | 2,1 | 2,2 |
| Val | 1,7 | 1,4 | 1,46 | 1,6 | 1,3 | 1,1 | 1 | 2,2 | 1,2 |
| Met | 0 | 0 | 0 | 0,8 | 0 | 0 | 0 | 0 | 0 |
| Ile | 1,63 | 1,2 | 1,3 | 1 | 2 | 2 | 2,1 | 1,1 | 1,9 |
| Leu | 2 | 2 | 2 | 2,1 | 2 | 2 | 2,1 | 2,1 | 2,1 |
| Phe | 1 | 1,3 | 1,3 | 1,6 | 2,3 | 1,5 | 1,6 | 1,1 | 1 |
| Lys | 1 | 1 | 1 | 1 | 1,1 | 0,94 | 1 | 1 | 1,1 |

## EXEMPLE 10:

On a préparé un soluté injectable répondant à la formulation suivante :
- Produit de l'exemple 1        1 mg
- excipient acqueux stérile        2 ml

## EXEMPLE 11:

On a prépare des comprimés répondant à la formule suivante :
- Produit de l'exemple 1     2 mg
- excipient q.s.p. un comprimé terminé à     150 mg (composition de l'excipient : lactose, amidon, talc, stéarate de magnésium).

## EXEMPLE 12:

On a préparé une pommade répondant à la formule suivante :
- Produit de l'exemple 1     10 mg
- excipient q.s.p.     100 mg

## ETUDE DE L'ACTIVITE ANTI-ELASTASE

L'activité anti-élastase a été déterminée par dosage spectrophotométrique vis à vis de l'élastase bovine et vis à vis de l'élastase leucocytaire humaine (par une technique analogue à celle décrite par Delmar et Tolia Biochem. 19, 468, 1980).

| Produits exemples | Elastase | Elastase leucocytaire humaine |
|---|---|---|
| 1 | $5 \times 10^{-6}\text{M}$ | $2 \times 10^{-9}\text{M}$ |
| 3 | $6 \times 10^{-7}\text{M}$ | $9 \times 10^{-8}\text{M}$ |
| 4 | $2,5 \times 10^{-8}\text{M}$ | $6 \times 10^{-8}\text{M}$ |
| 5 | | $2 \times 10^{-7}\text{M}$ |
| 6 | $3 \times 10^{-6}\text{M}$ | $2 \times 10^{-7}\text{M}$ |
| 7 | $8 \times 10^{-7}\text{M}$ | |
| 8 | | $2,5 \times 10^{-7}\text{M}$ |

## Revendications

**Revendications pour les Etats contractants suivants: AT, BE, CH, DE, DK, FR, GB, IT, LI, LU, NL, SE**

1. Micro-protéines renfermant 28 amino-acides répondant à la formule générale (I) :

G C P-X-Y-L-Z R C K Q D S D C L A G C V C G P N G F C G

     4 5   7    10     15     20     25

dans laquelle :
- X en position 4 représente un résidu de valine, de leucine, d'isoleucine, de norleucine, d'alanine, de cyclohexylalanine, d'o-méthylthéonine, de phénylglycine, d'acide alpha-amino butyrique ou d'arginine,
- Y en position 5 représente un résidu d'isoleucine ou de serine,
- Z en position 7 représente un résidu de méthionine ou de norleucine, étant entendu que Z représente un résidu de norleucine lorsque X représente un résidu d'arginine.

2. Microprotéines selon la revendication 1, caractérisées en ce que, Y en position 5 représente un résidu d'isoleucine, X en position 4 et Z en position 7 ont la signification déjà indiquée.

3. Microprotéines selon la revendication 1 ou 2, caractérisées en ce que Z en position 7 représente un résidu de norleucine.

4. Microprotéines selon l'une quelconque des revendications 1, 2 ou 3, caractérisées en ce que X en position 4 représente un résidu de valine, d'alanine ou de norleucine.

5. Microprotéine selon la revendication 1, caractérisée en ce que X en position 4 représente un résidu de valine, Y en position 5 représente un résidu d'isoleucine et Z en position 7 représente un résidu de norleucine.

6. Microprotéines selon la revendication 1, caractérisée en ce que X en position 4 représente un résidu d'alanine, Y en position 5 représente un résidu d'isoleucine et Z en position 7 représente un résidu de méthionine.

7. Microprotéines selon la revendication 1, caractérisée en ce que X en position 4 représente un résidu de norleucine, Y en position 5 représente un résidu de sérine et Z en position 7 représente un résidu de méthionine.

8. Procédé de préparation des nouvelles microprotéines telles que définies à la revendication 1, caractérisé en ce que l'on effectue une synthèse en phase solide en introduisant séquentiellement sur une résine oxyméthyl polystyrène réticulée les amino-acides dûment protégés en utilisant un agent de couplage, libère de la résine la chaîne peptidique ainsi formée et déprotège les amino-acides, puis, cyclise les 3 ponts disulfurés pour obtenir la microprotéine recherchée.

9. Procédé selon la revendication 8, caractérisé en ce que :
- la résine oxyméthyl polystyrène réticulée est une résine de type "Boc-gly-CM" dans laquelle Boc est un groupement tertiobutyloxycarbonyl, gly est un groupement glycine et CM désigne la résine oxyméthyl polystyrène réticulée,
- l'agent de couplage est le BOP ou (benzotriazol-1-yloxy) tris(diméthylamino) phosphonium hexafluorophosphate,
- la libération de la résine de la chaîne peptidique ainsi que la déprotection des amino-acdies est effectuée au moyen de l'acide fluorhydrique en opérant à basse température.

10. Médicaments, caractérisés en ce qu'ils sont constitués par les nouvelles microprotéines telles que définies par la formule générale (I) de la revendication 1.

11. Médicaments, caractérisés en ce qu'ils sont constitués par les nouvelles microprotéines telles que définies à l'une quelconque des revendications 2, 3, 4, 5, 6 ou 7.

12. Compositions pharmaceutiques, caractérisées en ce qu'elles renferment à titre de principe actif, l'un au moins des médicaments tels que définis à l'une quelconque des revendications 10 ou 11.

**Revendications pour l'Etat contractant suivant: ES**

1. Procédé de préparation des micro-protéines renfermant 28 amino-acides répondant à la formule générale (I) :

G C P-X-Y-L-Z R C K Q D S D C L A G C V C G P N G F C G

4 5 7 10 15 20 25

dans laquelle :

- X en position 4 représente un résidu de valine, de leucine, d'isoleucine, de norleucine, d'alanine, de cyclohexylalanine, d'o-méthylthéonine, de phénylglycine, d'acide alpha-amino butyrique ou d'arginine,
- Y en position 5 représente un résidu d'isoleucine ou de serine,
- Z en position 7 représente un résidu de méthionine ou de norleucine, étant entendu que Z représente un résidu de norleucine lorsque X représente un résidu d'arginine, caractérisé en ce que l'on effectue une synthèse en phase solide en introduisant séquentiellement sur une résine oxyméthyl polystyrène réticulée les amino-acides dûment protégés en utilisant un agent de couplage, libère de la résine la chaîne peptidique ainsi formée et déprotège les amino-acides, puis, cyclise les 3 ponts disulfurés pour obtenir la microprotéine recherchée.

2. Procédé selon la revendication 1, caractérisé en ce que :
- la résine oxyméthyl polystyrène réticulée est une résine de type "Boc-gly-CM" dans laquelle Boc est un groupement tertiobutyloxycarbonyl, gly est un groupement glycine et CM désigne la résine oxyméthyl polystyrène réticulée,
- l'agent de couplage est le BOP ou (benzotriazol-1-yloxy) tris(diméthylamino) phosphonium hexafluorophosphate,
- la libération de la résine de la chaîne peptidique ainsi que la déprotection des amino-acdies est effectuée au moyen de l'acide fluorhydrique en opérant à basse température.

3. Procédé selon la revendication 1 ou 2, caractérisé en ce que l'on introduit séquentiellement sur une résine oxyméthyle polystyrène réticulée les amino-acides tels que Y en position 5 représente un résidu d'isoleucine, X en position 4 et Z en position 7 ont la signification déjà indiquée.

4. Procédé selon l'une quelconque des revendications 1 à 3, caractérisé en ce que l'on introduit séquentiellement sur une résine oxyméthyle polystyrène réticulée les amino-acides tels que Z en position 7 représente un résidu de norleucine.

5. Procédé selon l'une quelconque des revendications 1 à 4, caractérisé en ce que l'on introduit séquentiellement sur une résine oxyméthyle polystyrène réticulée les amino-acides tels que X en position 4 représente un résidu de valine, d'alanine ou de norleucine.

6. Procédé selon l'une quelconque des revendications 1 à 5, caractérisé en ce que l'on introduit séquentiellement sur une résine oxyméthyle polystyrène réticulée les amino-acides tels que X en position 4 représente un résidu de valine, Y en position 5 représente un résidu d'isoleucine et Z en position 7 représente un résidu de norleucine.

7. Procédé selon l'une quelconque des revendications 1 à 6, caractérisé en ce que l'on introduit séquentiellement sur une résine oxyméthyle polystyrène réticulée les amino-acides tels que X en position 4 représente un résidu d'alanine, Y en position 5 représente un résidu d'isoleucine et Z en position 7 représente un résidu de méthionine.

8. Procédé selon l'une quelconque des revendications 1 à 7, caractérisé en ce que l'on introduit séquentiellement sur une résine oxyméthyle polystyrène réticulée les amino-acides tels que X en position 4 représente un résidu de norleucine, Y en position 5 représente un résidu de sérine et Z en position 7 représente un résidu de méthionine.

**Revendications pour l'Etat contractant suivant: GR**

1. Procédé de préparation des micro-protéines renfermant 28 amino-acides répondant à la formule générale (I) :

G C P-X-Y-L-Z R C K Q D S D C L A G C V C G P N G F C G

4 5 7 10 15 20 25

   dans laquelle :
   - X en position 4 représente un résidu de valine, de leucine, d'isoleucine, de norleucine, d'alanine, de cyclohexylalanine, d'o-méthylthéonine, de phénylglycine, d'acide alpha-amino butyrique ou d'arginine,
   - Y en position 5 représente un résidu d'isoleucine ou de serine,
   - Z en position 7 représente un résidu de méthionine ou de norleucine, étant entendu que Z représente un résidu de norleucine lorsque X représente un résidu d'arginine, caractérisé en ce que l'on effectue une synthèse en phase solide en introduisant séquentiellement sur une résine oxyméthyl polystyrène réticulée les amino-acides dûment protégés en utilisant un agent de couplage, libère de la résine la chaîne peptidique ainsi formée et déprotège les amino-acides, puis, cyclise les 3 ponts disulfurés pour obtenir la microprotéine recherchée.

2. Procédé selon la revendication 1, caractérisé en ce que :
   - la résine oxyméthyl polystyrène réticulée est une résine de type "Boc-gly-CM" dans laquelle Boc est un groupement tertiobutyloxycarbonyl, gly est un groupement glycine et CM désigne la résine oxyméthyl polystyrène réticulée,
   - l'agent de couplage est le BOP ou (benzotriazol-1-yloxy) tris(diméthylamino) phosphonium hexafluorophosphate,
   - la libération de la résine de la chaîne peptidique ainsi que la déprotection des amino-acdies est effectuée au moyen de l'acide fluorhydrique en opérant à basse température.

3. Procédé selon la revendication 1 ou 2, caractérisé en ce que l'on introduit séquentiellement sur une résine oxyméthyle polystyrène réticulée les amino-acides tels que Y en position 5 représente un résidu d'isoleucine, X en position 4 et Z en position 7 ont la signification déjà indiquée.

4. Procédé selon l'une quelconque des revendications 1 à 3, caractérisé en ce que l'on introduit séquentiellement sur une résine oxyméthyle polystyrène réticulée les amino-acides tels que Z en position 7 représente un résidu de norleucine.

5. Procédé selon l'une quelconque des revendications 1 à 4, caractérisé en ce que l'on introduit séquentiellement sur une résine oxyméthyle polystyrène réticulée les amino-acides tels que X en position 4 représente un résidu de valine, d'alanine ou de norleucine.

6. Procédé selon l'une quelconque des revendications 1 à 5, caractérisé en ce que l'on introduit séquentiellement sur une résine oxyméthyle polystyrène réticulée les amino-acides tels que X en position 4 représente un résidu de valine, Y en position 5 représente un résidu d'isoleucine et Z en position 7 représente un résidu de norleucine.

7. Procédé selon l'une quelconque des revendications 1 à 6, caractérisé en ce que l'on introduit séquentiellement sur une résine oxyméthyle polystyrène réticulée les amino-acides tels que X en position 4 représente un résidu d'alanine, Y en position 5 représente un résidu d'isoleucine et Z en position 7 représente un résidu de méthionine.

8. Procédé selon l'une quelconque des revendications 1 à 7, caractérisé en ce que l'on introduit séquentiellement sur une résine oxyméthyle polystyrène réticulée les amino-acides tels que X en position 4 représente un résidu de norleucine, Y en position 5 représente un résidu de sérine et Z en position 7 représente un résidu de méthionine.

9. Procédé de préparation de compositions pharmaceutiques, caractérisé en ce que l'on met à titre de principe actif l'une au moins des microprotéines de formule (I) telle que définie à la revendication 1 sous une forme destinée à cet usage.

10. Procédé de préparation de compositions pharmaceutiques, caractérisé en ce que l'on met à titre de principe actif l'une au moins des microprotéines de formule (I) telle que définie à l'une quelconque des revendications 2 à 8, sous une forme desinée à cet usage.

## Claims

### Claims for the following Contracting States : AT, BE, CH, DE, DK, FR, GB, IT, LI, LU, NL, SE

1. Microproteins containing 28 amino acids corresponding to the general formula (I):

$$G \quad C \quad P–\underline{X}–\underline{Y}–L–\underline{Z} \quad R \quad C \quad K \quad Q \quad D \quad S \quad D \quad C \quad L \quad A \quad G \quad C \quad V \quad C \quad G \quad P \quad N \quad G \quad F \quad C \quad G$$

$$4 \quad 5 \quad\quad 7 \quad\quad 10 \quad\quad\quad 15 \quad\quad\quad 20 \quad\quad\quad 25$$

in which:
- X in position 4 represents a residue of one of the following: valine, leucine, isoleucine, norleucine, alanine, cyclohexylalanine, o-methyltheonine, phenylglycine, alpha-amino butyric acid or arginine,
- Y in position 5 represents an isoleucine or serine residue,
- Z in position 7 represents a methionine or norleucine residue, it being understood that Z represents a norleucine residue when X represents an arginine residue.

2. Microproteins according to claim 1, characterized in that Y in position 5 represents an isoleucine residue, X in position 4 and Z in position 7 have the meaning already indicated.

3. Microproteins according to claim 1 or 2, characterized in that Z in position 7 represents a norleucine residue.

4. Microproteins according to any one of claims 1, 2 or 3, characterized in that X in position 4 represents a valine, alanine or norleucine residue.

5. Microprotein according to claim 1, characterized in that X in position 4 represents a valine residue, Y in position 5 represents an isoleucine residue and Z in position 7 represents a norleucine residue.

6. Microproteins according to claim 1, characterized in that X in position 4 represents an alanine residue, Y in position 5 represents an isoleucine residue and Z in position 7 represents a methionine residue.

7. Microproteins according to claim 1, characterized in that X in position 4 represents a norleucine residue, Y in position 5 represents a serine residue and Z in position 7 represents a methionine residue.

8. Preparation process for new micro proteins as defined in claim 1, characterized in that a solid phase synthesis is carried out by sequentially introducing the duly protected amino acids on a cross-linked oxymethyl polystyrene resin using a coupling agent, the peptide chain thus formed is released from the resin and the amino acids are deprotected, then the 3 disulphide bridges are cyclized in order to obtain the desired microprotein.

9. Process according to claim 8, characterized in that:
- the cross-linked oxymethyl polystyrene resin is a resin of "Boc-gly-CM" type in which Boc is a tertiobutyloxycarbonyl group, gly is a glycine group and Cm designates the crosslinked oxymethyl polystyrene resin,
- the coupling agent is BOP or (benzotriazol-1-yloxy) tris(dimethylamino) phosphonium hexafluorophosphate,
- the release of the peptide chain from the resin as well as the deprotection of the amino acids is carried out using hydrofluoric acid operating at low temperature.

10. Medicaments, characterized in that they are constituted by the new microproteins as defined by general formula (I) of claim 1.

11. Medicaments, characterized in that they are constituted by new microproteins as defined in any one of claims 2, 3, 4, 5, 6 or 7.

12. Pharmaceutical compositions, characterized in that they contain as active ingredient at least one of the medicaments as defined in any one of claims 10 or 11.

**Claims for the following Contracting State : ES**

1. Preparation process for microproteins containing 28 amino acids corresponding to general formula (I):

G C P–$\underline{X}$–$\underline{Y}$–L–$\underline{Z}$ R C K Q D S D C L A G C V C G P N G F C G

4 5    7    10    15    20    25

in which:
- X in position 4 represents a residue of one of the following: valine, leucine, isoleucine, norleucine, alanine, cyclohexylalanine, o-methyltheonine, phenylglycine, alpha-amino butyric acid or arginine,
- Y in position 5 represents an isoleucine or serine residue,
- Z in position 7 represents a methionine or norleucine residue, it being understood that Z represents a norleucine residue when X represents an arginine residue, characterized in that a solid phase synthesis is carried out by sequentially introducing the duly-protected amino acids on a cross-linked oxymethyl polystyrene resin using a coupling agent, the peptide chain thus formed is released from the resin and the amino acids are deprotected, then the 3 disulphide bridges are cyclized in order to obtain the desired microprotein.

2. Process according to claim 1, characterized in that:
- the cross-linked oxymethyl polystyrene resin is a resin of "Boc-gly-CM" type in which Boc is a tertiobutyloxycarbonyl group, gly is a glycine group and Cm designates the crosslinked oxymethyl polystyrene resin,
- the coupling agent is BOP or (benzotriazol 1-yloxy) tris(dimethylamino) phosphonium hexafluorophosphate,
- the release of the peptide chain from the resin as well as the deprotection of the amino acids is carried out using hydrofluoric acid operating at low temperature.

3. Process according to claim 1 or 2, characterized in that the amino acids are sequentially introduced on a cross-linked oxymethyl polystyrene resin such that Y in position 5 represents an isoleucine residue, X in position 4 and Z in position 7 have the meaning already indicated.

4. Process according to any one of claims 1 to 3, characterized in that the amino acids are sequentially introduced on a cross-linked oxymethyl polystyrene resin such that Z in position 7 represents a norleucine residue.

5. Process according to any one of claims 1 to 4, characterized in that the amino acids are sequentially introduced on a cross-linked oxymethyl polystyrene resin such that X in position 4 represents a valine, alanine or norleucine residue.

6. Process according to any one of claims 1 to 5, characterized in that the amino acids are sequentially introduced on a cross-linked oxymethyl polystyrene resin such that X in position 4 represents a valine residue, Y in position 5 represents an isoleucine residue and Z in position 7 represents a norleucine residue.

7. Process according to any one of claims 1 to 6, characterized in that the amino acids are sequentially introduced on a cross-linked oxymethyl polystyrene resin such that X in position 4 represents an alanine residue, Y in position 5 represents an isoleucine residue and Z in position 7 represents a methionine residue.

8. Process according to any one of claims 1 to 7, characterized in that the amino acids are sequentially introduced on a cross-linked oxymethyl polystyrene resin such that X in position 4 represents a norleucine residue, Y in position 5 represents a serine residue and Z in position 7 represents a methionine residue.

**Claims for the following Contracting State : GR**

1. Preparation process for microproteins containing 28 amino acids corresponding to general formula (I):

$$\text{G C P-}\underline{X}\text{-}\underline{Y}\text{-L-}\underline{Z}\text{ R C K Q D S D C L A G C V C G P N G F C G}$$

4 5   7     10       15       20       25

in which:
- X in position 4 represents a residue of one of the following: valine, leucine, isoleucine, norleucine, alanine, cyclohexylalanine, o-methyltheonine, phenylglycine, alpha-amino butyric acid or arginine,
- Y in position 5 represents an isoleucine or serine residue,
- Z in position 7 represents a methionine or norleucine residue, it being understood that Z represents a norleucine residue when X represents an arginine residue, characterized in that a solid phase synthesis is carried out by sequentially introducing the duly-protected amino acids on a cross-linked oxymethyl polystyrene resin using a coupling agent, the peptide chain thus formed is released from the resin and the amino acids are deprotected, then the 3 disulphide bridges are cyclized in order to obtain the desired microprotein.

2. Process according to claim 1, characterized in that:
- the cross-linked oxymethyl polystyrene resin is a resin of "Boc-gly-CM" type in which Boc is a tertiobutyloxycarbonyl group, gly is a glycine group and Cm designates the crosslinked oxymethyl polystyrene resin,
- the coupling agent is BOP or (benzotriazol 1-yloxy) tris(dimethylamino) phosphonium hexafluorophosphate,
- the release of the peptide chain from the resin as well as the deprotection of the amino acids is carried out using hydrofluoric acid operating at low temperature.

3. Process according to claim 1 or 2, characterized in that the amino acids are sequentially introduced on a cross-linked oxymethyl polystyrene resin such that Y in position 5 represents an isoleucine residue, X in position 4 and Z in position 7 have the meaning already indicated.

4. Process according to any one of claims 1 to 3, characterized in that the amino acids are sequentially introduced on a cross-linked oxymethyl polystyrene resin such that Z in position 7 represents a norleucine residue.

5. Process according to any one of claims 1 to 4, characterized in that the amino acids are sequentially introduced on a cross-linked oxymethyl polystyrene resin such that X in position 4 represents a valine, alanine or norleucine residue.

6. Process according to any one of claims 1 to 5, characterized in that the amino acids are sequentially in-

12

troduced on a cross-linked oxymethyl polystyrene resin such that X in position 4 represents a valine residue, Y in position 5 represents an isoleucine residue and Z in position 7 represents a norleucine residue.

7. Process according to any one of claims 1 to 6, characterized in that the amino acids are sequentially introduced on a cross-linked oxymethyl polystyrene resin such that X in position 4 represents an alanine residue, Y in position 5 represents an isoleucine residue and Z in position 7 represents a methionine residue.

8. Process according to any one of claims 1 to 7, characterized in that the amino acids are sequentially introduced on a cross-linked oxymethyl polystyrene resin such that X in position 4 represents a norleucine residue, Y in position 5 represents a serine residue and Z in position 7 represents a methionine residue.

9. Preparation process for pharmaceutical compositions, characterized in that at least one of the microproteins of formula (I) as defined in claim 1 is used as active ingredient in a form intended for this use.

10. Preparation process for pharmaceutical compositions, characterized in that at least one of the microproteins of formula (I) as defined in any one of claims 2 to 8 is used as active ingredient in a form intended for this use.

**Patentansprüche**

**Patentanprüche für folgende Vertragsstaaten : AT, BE, CH, DE, DK, FR, GB, IT, LI, LU NL SE**

1. Mikroproteine mit 28 Aminosauren der allgemeinen Formel (I)

```
G  C  P-X̲-Y̲-L-Z̲  R  C  K  Q  D  S  D  C  L  A  G  C  V  C  G  P  N  G  F  C  G
      4  5    7        10          15          20              25
```

worin
- X in Position 4 für einen Valin-, Leucin-, Isoleucin-, Norleucin-, Alanin-, Cyclohexylalanin-, O-Methyl-threonin-, Phenylglycin-, $\alpha$-Aminobuttersäure- oder Argininrest steht,
- Y in Position 5 für einen Isoleucin- oder Serinrest steht,
- Z in Position 7 für einen Methionin- oder Norleucinrest steht, mit der Maßgabe, daß Z für einen Norleucinrest steht, wenn X für einen Argininrest steht.

2. Mikroproteine nach Anspruch 1, **dadurch gekennzeichet, daß** Y in Position 5 für einen Isoleucinrest steht, X in Position 4 und Z in Position 7 wie vorstehend definiert sind.

3. Mikroproteine nach Anspruch 1 oder 2, **dadurch gekennzeichet, daß** Z in Position 7 fur einen Norleucinrest steht.

4. Mikroproteine nach einem der Ansprüche 1, 2 oder 3, **dadurch gekennzeichet, daß** X in Position 4 für einen Valin-, Alanin- oder Norleucinrest steht.

5. Mikroproteine nach Anspruch 1, **dadurch gekennzeichet, daß** X in Position 4 für einen Valinrest steht, Y in Position 5 für einen Isoleucinrest steht, und Z in Position 7 für einen Norleucinrest steht.

6. Mikroproteine nach Anspruch 1, **dadurch gekennzeichet, daß** X in Position 4 für einen Alaninrest steht, Y in Position 5 für einen Isoleucinrest steht, und Z in Position 7 für einen Methioninrest steht.

**7.** Mikroproteine nach Anspruch 1, **dadurch gekennzeichet, daß** X in Position 4 für einen Norleucinrest steht, Y in Position 5 für einen Serinrest steht, und Z in Position 7 für einen Methioninrest steht.

**8.** Verfahren zur Herstellung der neuen in Anspruch 1 definierten Mikroproteine, **dadurch gekennzeichet, daß** man eine Festphasensynthese durchführt, indem man stufenweise an einem vernetzten Oxymethylpolystyrolharz die geeigneterweise geschützten Aminosäuren unter Verwendung eines Kupplungsmittels einführt, die so gebildete Peptidkette von dem Harz freisetzt und die Schutzgruppen von den Aminosäuren abspaltet, anschließend die 3 Disulfidbrücken zyklisiert, um das gesuchte Mikroprotein zu erhalten.

**9.** Verfahren nach Anspruch 8, **dadurch gekennzeichnet, daß**
- das vernetzte Oxymethylpolystyrolharz ein Harz vom Typ "Boc-Gly-CM" ist, worin Boc eine tert.-Butyloxycarbonylgruppe ist, Gly ein Glycinrest ist, und CM das vernetzte Oxymethylpolystyrolharz bedeutet,
- das Kupplungsmittel BOP oder Tris-(dimethylamino)-benzotriazol-1-yloxyphosphoniumhexafluorphosphat ist,
- die Freisetzung der Peptidkette von dem Harz, sowie die Abspaltung der Schutzgruppen von den Aminosäuren mittels Fluorwasserstoffsäure bei einer tiefen Temperatur durchgeführt wird.

**10.** Medikamente, **dadurch gekennzeichnet, daß** sie die neuen Mikroproteine gemäß der allgemeinen Formel (I) nach Anspruch 1 enthalten.

**11.** Medikamente, **dadurch gekennzeichet, daß** sie die neuen Mikroproteine nach einem der Ansprüche 2, 3, 4, 5, 6 oder 7 enthalten.

**12.** Pharmazeutische Präparate, **dadurch gekennzeichet, daß** sie als Wirkstoff mindestens eines der Medikamente nach einem der Ansprüche 10 oder 11 enthalten.

**Patentansprüche für folgenden Vertragsstaat : ES**

**1.** Verfahren zur Herstellung von Mikroproteinen mit 28 Aminosäuren der allgemeinen Formel (I)

G  C  P-X̲-Y̲-L-Z̲  R  C  K  Q  D  S  D  C  L  A  G  C  V  C  G  P  N  G  F  C  G

  4  5     7       10         15         20              25

worin
- X in Position 4 für einen Valin-, Leucin-, Isoleucin-, Norleucin-, Alanin-, Cyclohexylalanin-, O-Methylthreonin-, Phenylglycin-, α-Aminobuttersäure- oder Argininrest steht,
- Y in Position 5 für einen Isoleucin- oder Serinrest steht,
- Z in Position 7 für einen Methionin- oder Norleucinrest steht, mit der Maßgabe, daß Z für einen Norleucinrest steht, wenn X für einen Argininrest steht, **dadurch gekennzeichet, daß** man eine Festphasensynthese durchführt, indem man stufenweise an einem vernetzten Oxymethylpolystyrolharz die geeigneterweise geschützten Aminosäuren unter Verwendung eines Kupplungsmittels einführt, die so gebildete Peptidkette von dem Harz freisetzt und die Schutzgruppen von den Aminosäuren abspaltet, anschließend die 3 Disulfidbrücken zyklisiert, um das gesuchte Mikroprotein zu erhalten.

**2.** Verfahren nach Anspruch 1, **dadurch gekennzeichet, daß**
- das vernetzte Oxymethylpoiystyrolharz ein Harz vom Typ "Boc-Gly-CM" ist, worin Boc eine tert.- Butyloxycarbonylgruppe ist, Gly ein Glycinrest ist, und CM das vernetzte Oxymethylpolystyrolharz bedeutet,
- das Kupplungsmittel BOP oder Tris-(dimethylamino)- benzotriazol-1-yloxyphosphoniumhexafluor-

14

phosphat ist,
- die Freisetzung der Peptidkette von dem Harz, sowie die Abspaltung der Schutzgruppen von den Aminosäuren mittels Fluorwasserstoffsäure bei einer tiefen Temperatur durchgeführt wird.

3. Verfahren nach Anspruch 1 oder 2, **dadurch gekennzeichet, daß** man stufenweise auf einem vernetzten Oxymethylpolystyrolharz Aminosäuren einführt, bei denen Y in Position 5 für einen Isoleucinrest steht, X in Position 4 und Z in Position 7 wie vorstehend definiert sind.

4. Verfahren nach einem der Ansprüche 1 bis 3, **dadurch gekennzeichet, daß** man stufenweise auf einem vernetzten Oxymethylpolystyrolharz Aminosäuren einführt, bei denen Z in Position 7 für einen Norleucinrest steht.

5. Verfahren nach einem der Ansprüche 1 bis 4, **dadurch gekenn- zeichnet, daß** man stufenweise auf einem vernetzten Oxymethylpolystyrolharz Aminosäuren einführt, bei denen X in Position 4 für einen Valin-, Alanin- oder Norleucinrest steht.

6. Verfahren nach einem der Ansprüche 1 bis 5, **dadurch gekenn- zeichnet, daß** man stufenweise auf einem vernetzten Oxymethylpolystyrolharz Aminosäuren einführt, bei denen X in Position 4 für einen Valinrest steht, Y in Position 5 für einen Isoleucinrest steht, und Z in Position 7 für einen Norleucinrest steht.

7. Verfahren nach einem der Ansprüche 1 bis 6, **dadurch gekennzeichet, daß** man stufenweise auf einem vernetzten Oxymethylpolystyrolharz Aminosäuren einführt, bei denen X in Position 4 für einen Alaninrest steht, Y in Position 5 für einen Isoleucinrest steht, und Z in Position 7 für einen Methioninrest steht.

8. Verfahren nach einem der Ansprüche 1 bis 7, **dadurch gekennzeichet, daß** man stufenweise auf einem vernetzten Oxymethylpolystyrolharz Aminosäuren einführt, bei denen X in Position 4 für einen Norleucinrest steht, Y in Position 5 für einen Serinrest steht, und Z in Position 7 für einen Methioninrest steht.

**Patentansprüche für folgenden Vertragsstaat : GR**

1. Verfahren zur Herstellung von Mikroproteinen mit 28 Aminosäuren der allgemeinen Formel (I)

G  C  P-X̲-Y̲-L-Z̲  R  C  K  Q  D  S  D  C  L  A  G  C  V  C  G  P  N  G  F  C  G

4  5      7          10          15          20          25

worin
- X in Position 4 für einen Valin-, Leucin-, Isoleucin-, Norleucin-, Alanin-, Cyclohexylalanin-, O-Methyl-threonin-, Phenylglycin-, $\alpha$-Aminobuttersäure- oder Argininrest steht,
- Y in Position 5 für einen Isoleucin- oder Serinrest steht,
- Z in Position 7 für einen Methionin- oder Norleucinrest steht, mit der Maßgabe, daß Z für einen Norleucinrest steht, wenn X für einen Argininrest steht, **dadurch gekennzeichet, daß** man eine Festphasensynthese durchführt, indem man stufenweise an einem vernetzten Oxymethylpolystyrolharz die geeigneterweise geschützten Aminosäuren unter Verwendung eines Kupplungsmittels einführt, die so gebildete Peptidkette von dem Harz freisetzt und die Schutzgruppen von den Aminosäuren abspaltet, anschließend die 3 Disulfidbrücken zyklisiert, um das gesuchte Mikroprotein zu erhalten.

2. Verfahren nach Anspruch 1, **dadurch gekennzeichet, daß**
- das vernetzte Oxymethylpolystyrolharz ein Harz vom Typ "Boc-Gly-CM" ist, worin Boc eine tert.-Butyloxycarbonylgruppe ist, Gly ein Glycinrest ist und CM das vernetzte Oxymethylpolystyrolharz bedeutet,

- das Kupplungsmittel BOP oder Tris-(dimethylamino)-benzotriazol-1-yloxyphosphoniumhexafluor-phosphat ist,
- die Freisetzung der Peptidkette von dem Harz, sowie die Abspaltung der Schutzgruppen von den Aminosäuren mittels Fluorwasserstoffsäure bei einer tiefen Temperatur durchgeführt wird.

3. Verfahren nach Anspruch 1 oder 2, **dadurch gekennzeichet, daß** man stufenweise auf einem vernetzten Oxymethylpolystyrolharz Aminosäuren einführt, bei denen Y in Position 5 für einen Isoleucinrest steht, X in Position 4 und Z in Position 7 wie vorstehend definiert sind.

4. Verfahren nach einem der Ansprüche 1 bis 3, **dadurch gekennzeichet, daß** man stufenweise auf einem vernetzten Oxymethylpolystyrolharz Aminosäuren einführt, bei denen Z in Position 7 für einen Norleucinrest steht.

5. Verfahren nach einem der Ansprüche 1 bis 4, **dadurch gekennzeichet, daß** man stufenweise auf einem vernetzten Oxymethylpolystyrolharz Aminosäuren einführt, bei denen X in Position 4 für einen Valin-, Alanin- oder Norleucinrest steht.

6. Verfahren nach einem der Ansprüche 1 bis 5, **dadurch gekennzeichet, daß** man stufenweise auf einem vernetzten Oxymethylpolystyrolharz Aminosäuren einführt, bei denen X in Position 4 für einen Valinrest steht, Y in Position 5 für einen Isoleucinrest steht, und Z in Position 7 für einen Norleucinrest steht.

7. Verfahren nach einem der Ansprüche 1 bis 6, **dadurch gekennzeichet, daß** man stufenweise auf einem vernetzten Oxymethylpolystyrolharz Aminosäuren einführt, bei denen X in Position 4 für einen Alaninrest steht, Y in Position 5 für einen Isoleucinrest steht, und Z in Position 7 für einen Methioninrest steht.

8. Verfahren nach einem der Ansprüche 1 bis 7, **dadurch gekennzeichet, daß** man stufenweise auf einem vernetzten Oxymethylpolystyrolharz Aminosäuren einführt, bei denen X in Position 4 für einen Norleucinrest steht, Y in Position 5 für einen Serinrest steht, und Z in Position 7 für einen Methioninrest steht.

9. Verfahren zur Herstellung von pharmazeutischen Präparaten, **dadurch gekennzeichet, daß** man als Wirkstoff mindestens eines der Mikroproteine der Formel (I) nach Anspruch 1 in einer für diese Verwendung geeigneten Form verwendet.

10. Verfahren zur Herstellung von pharmazeutischen Präparaten, **dadurch gekennzeichet, daß** man als Wirkstoff mindestens eines der Mikroproteine der Formel (I) nach einem der Ansprüche 2 bis 8 in einer für diese Verwendung geeigneten Form verwendet.